# EUROPÄISCHE PATENTANMELDUNG

(11) **EP 1 914 231 A2**
(43) Veröffentlichungstag der Anmeldung: **23.04.2008**
(21) Anmeldenummer: 07116310.9
(22) Anmeldetag: 13.09.2007
(51) Int. Cl.: C07D 319/06, C11B 9/00

(54) **2-Methyl-2-alkenyl-substituierte 1,3-Dioxane als Riechstoffe**

(30) Priorität: 16.09.2006 DE 102006043587
(71) Anmelder: Symrise GmbH & Co. KG, 37603 Holzminden (DE)
(72) Erfinder: Bertram, Heinz-Jürgen, 37603 Holzminden (DE); Obrocki, Thomas, 30982 Pattensen (DE); Lüders, Sonja, 37603 Holzminden (DE)
(74) Vertreter: Eisenführ, Speiser & Partner

(57) **Zusammenfassung**

Die vorliegende Erfindung betrifft eine Verbindung der Formel (I) 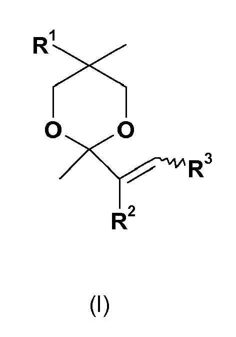 wobei
R¹ Wasserstoff oder Methyl bedeutet, und R² und R³ unabhängig voneinander Methyl oder Ethyl bedeuten,
ein Verfahren zu ihrer Herstellung und die Verwendung dieser Verbindungen als Riechstoff.

## Beschreibung

Die vorliegende Erfindung betrifft primär bestimmte neue Verbindungen der Formel (I) (2-Methyl-2-alkenyl-substituierte 1,3-Dioxane), ein Verfahren zu ihrer Herstellung und die Verwendung dieser Verbindungen als Riechstoff. Daneben betrifft die Erfindung u. a. parfümierte Produkte wie Riechstoffmischungen (Riechstoffkompositionen) umfassend eine (sensorisch wirksame) Menge dieser Verbindungen. Weitere Aspekte der Erfindung ergeben sich aus der nachfolgenden Beschreibung und den beigefügten Patentansprüchen.

Die neuen Verbindungen (2-Methyl-2-alkenyl-substituierte 1,3-Dioxane) der Formel (I) haben folgende Struktur: 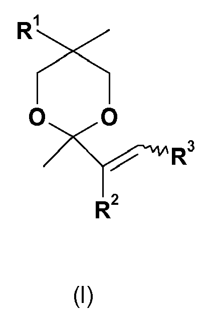 wobei
R¹ Wasserstoff oder Methyl bedeutet, und
R² und R³ unabhängig voneinander Methyl oder Ethyl bedeuten.

Die geschlängelte Linie in der obigen und den weiteren folgenden Strukturformeln bedeutet, dass die Doppelbindung in (E)- oder (Z)-Konfiguration vorliegen kann.

In der Parfümindustrie besteht generell ein Bedarf an neuartigen und originellen Riechstoffen, da den Konsumenten laufend neue und moderne Düfte zur Verfügung gestellt werden sollen. Wegen der steigenden Nachfrage der Verbraucher nach neuen modernen Duftnoten, besteht in der Parfümindustrie ein ständiger Bedarf an neuen Duftstoffen, mit denen sich in Parfüms neuartige Effekte erzielen und auf diese Art neue Modetrends kreieren lassen.

Für die Kreation neuartiger moderner Kompositionen besteht ständiger Bedarf an neuen Riechstoffen mit besonderen geruchlichen Eigenschaften, die geeignet sind, als Grundlage für die Komposition von neuartigen modernen Parfüms zu dienen. Die gesuchten Riechstoffe sollen neben einem typischen Primärgeruch weitere Noten und Aspekte aufweisen, die ihnen geruchlichen Charakter, wie beispielsweise Frische und Komplexität verleihen.

Es war daher die Aufgabe der vorliegende Erfindung, Riechstoffe mit neuen frischen Geruchseigenschaften zu finden, mit welchen Riechstoffkompositionen besondere geruchliche Noten und Aspekte verliehen werden können.

Von großem Interesse für die parfümistische Komposition sind daneben Riechstoffe, die die Stärke der geruchlichen Wahrnehmung anderer Riechstoffe erhöhen (also als Booster fungieren).

Die Suche nach geeigneten Riechstoffen, die zur vorliegenden Erfindung führte, wurde durch folgende Sachverhalte erschwert:
- Die Mechanismen der Geruchswahrnehmung sind nicht ausreichend bekannt.
- Die Zusammenhänge zwischen der speziellen Geruchswahrnehmung einerseits und der chemischen Struktur des zugehörigen Riechstoffs andererseits sind nicht hinreichend erforscht.
- Häufig bewirken bereits geringfügige Änderungen am strukturellen Aufbau eines bekannten Riechstoffs starke Änderungen der sensorischen Eigenschaften und beeinträchtigen die Verträglichkeit für den menschlichen Organismus.

Der Erfolg der Suche nach geeigneten Riechstoffen hängt deshalb stark von der Intuition des Suchenden ab.

Es wurde nun überraschenderweise gefunden, dass 2-Methyl-2-alkenyl-substituierte 1,3-Dioxane der obigen Formel (I) geeignet sind, die gestellte Aufgabe zu lösen.

Nach Meinung der Parfümeure weisen die erfindungsgemäßen 2-Methyl-2-alkenyl-substituierte 1,3-Dioxane der Formel (I) folgende komplexe Geruchscharakterisitk auf:
minzig, camphrig, damasconig, fruchtig, Rumrosine, Trockenfrucht, süß, grün.

Unter "Rumrosine" wird generell der alkoholische Geruch von in Rum eingelegten Rosinen verstanden.

"Minzig" kann in Abhängigkeit von der jeweiligen Bedeutung der Reste R¹, R² und R³ etwas variieren von Pfefferminze bis Krauseminze (Spearmint), in manchen Fällen gepaart mit einer leichten Eucalyptus-Note.

Die begehrte damasconige Note der erfindungsgemäßen Verbindungen ähnelt der des alpha-Damascons am meisten.

Es hat sich überraschenderweise gezeigt, dass sich die 2-Methyl-2-alkenyl-substituierte 1,3-Dioxane der Formel (I) von in der Literatur beschriebenen, strukturell verwandten Verbindungen (substituierte 1,3-Dioxane), geruchlich deutlich unterscheidet.

EP 0 039 029 beschreibt 2-(1'-Methylbutyl)-1,3-dioxane, die in der vierten und fünften Position mit 2 bis 6 Alkylgruppen substituiert sind. Diesen Verbindungen wird eine blumige und fruchtige Note zugeschrieben.

US 4,146,506 betrifft Parfumkompositionen mit 4-Isopropyl-5,5-dimethyl-1,3-dioxan mit C1-C3 Alkylsubstituenten in der zweiten Position. Diese 1,3-Dioxane werden mit einer fruchtigen, krautigen und holzigen Note beschrieben.

US 5,888,961 beschreibt 2-Isobutyl-5-methyl-1,3-dioxan mit einem starken Geruchseindruck nach Kamille sowie fruchtigen, anisigen, minzigen, camphrigen und grünen Noten.

CH 592649 betrifft 2-(2'-Methyl-1'-propenyl)-1,3-dioxane mit einer oder mehreren Methyl- oder Ethylgruppen in der vierten und/oder fünften und/oder sechsten Position. Diesen wird ein rosenartiger Geruchseindruck, ähnlich Rosenoxid, zugeschrieben. Den erfindungsgemäßen Verbindungen der Formel (I) am strukturell ähnlichsten in CH 592649 ist 5,5-Dimethyl-2-(2'-methyl-1'-propenyl)-1,3-dioxan, welches laut CH 592649 einen Thymian-Geruch aufweist.

In Pishch. Prom-st. (Moscow) (1990), (2), 54-57 wird 2-Isobutyl-2,5,5-trimethyl-1,3-dioxan als Verbindung mit einem holzig-fruchtigen Geruch mit Lösungsmittelnote beschrieben.

Die in der Literatur bislang beschriebenen Riechstoffe mit substituierter 1,3-Dioxan - Struktur sind Riechstoffe, die im Vergleich mit den erfindungsgemäßen Verbindungen der Formel (I)
- andere geruchliche Eigenschaften haben, und/oder
- sich deutlich hinsichtlich ihres Substitutionsmusters in den Positionen 2, 4, 5 und/oder 6 unterscheiden, und/oder
- sich hinsichtlich der Kettenlänge und/oder Struktur der Seitenkette an Position 2 unterscheiden.

Zudem sind 1,3-Dioxane, die an Position 2 nur einfach substituiert sind (d.h. nicht-erfindungsgemäße Verbindungen, die sich von Aldehyden ableiten), weniger stabil als an Position 2 disubstituierte Verbindungen (wie z. B. die erfindungsgemäßen und andere Verbindungen, welche sich von Ketonen ableiten).

In eigenen Untersuchungen wurde zudem gefunden, dass im Vergleich mit erfindungsgemäßen Verbindungen bei Abwesenheit eines Substituenten an Position 5 der Geruchseindruck stark verändert ist.

So riecht beispielsweise die nicht-erfindungsgemäße Verbindung 2-Methyl-2-(1-methyl-propenyl)-1,3-dioxan im Gegensatz zu den erfindungsgemäßen Verbindungen der Formel (I) fischig, walnussig, krautig, blumig, mit Noten von Lorbeer, Lavendel und Linalool.

Bevorzugte Verbindungen der Formel (I) sind solche mit R² = Methyl, d.h. Verbindungen der Formel (II): 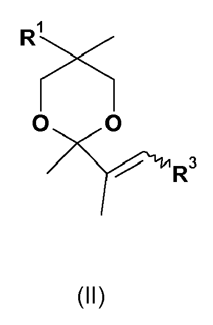 wobei
R¹ Wasserstoff oder Methyl bedeutet, und
R³ bedeutet Methyl oder Ethyl.

Bevorzugt sind erfindungsgemäße Verbindungen der Formel (I) und insbesondere erfindungsgemäße Verbindungen der Formel (II), in denen die Doppelbindung (E)-Konfiguration aufweist.

Besonders bevorzugte erfindungsgemäße Verbindungen der Formel (I) sind solche mit R² = Methyl und R³ = Methyl, wobei die Doppelbindung vorzugsweise (E)-Konfiguration aufweist. Diese besonders bevorzugten erfindungsgemäßen Verbindungen sind solche der Formel (III). 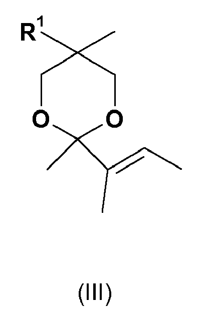 wobei
R¹ Wasserstoff oder Methyl bedeutet.

Die erfindungsgemäßen (E)-Isomere der Formel (III) zeigen einen besonders stark ausgeprägten und sehr natürlichen, hellen und transparenten Geruch.

Die erfindungsgemäßen Verbindungen der Formel (I) und insbesondere die erfindungsgemäßen Verbindungen der Formeln (II) und (III), besonders bevorzugt die erfindungsgemäßen Verbindungen mit (E)-Konfiguration an der Doppelbindung, können für den Fall, dass R¹ Wasserstoff bedeutet als cis- oder trans-Diastereomer oder auch als beliebiges Gemisch dieser Diastereomeren vorliegen.

In Mischungen mit anderen Riechstoffen vermögen die erfindungsgemäßen Verbindungen der Formel (I) bereits in geringer Dosierung die Intensität einer Riechstoffmischung zu verstärken und das Gesamtbild der Riechstoffmischung geruchlich abzurunden sowie der Mischung mehr Ausstrahlung und Natürlichkeit zu verleihen. In höheren Dosierungen kommt besonders der frische und damasconige Geruch zum Tragen.

Die vorliegende Erfindung betrifft auch Mischungen umfassend oder bestehend aus:
(a) einer oder mehreren erfindungsgemäßen Verbindungen der Formel (I), vorzugsweise jedoch der Formel (II), besonders bevorzugt der Formel (III), ganz besonders bevorzugt mit (E)-Konfiguration an der Doppelbindung,
   und
(b) 2-Ethyl-2,5,5-trimethyl 1,3-Dioxan der Formel (IV) 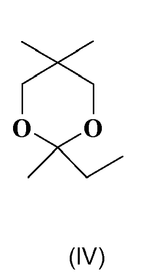 und/oder
(c) 3-Methyl-3-penten-2-on der Formel (V) 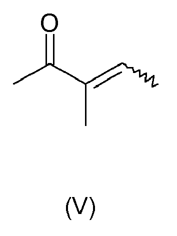
sowie gegebenenfalls ein, zwei oder mehr weiteren Riechstoffen.

2-Ethyl-2,5,5-trimethyl-1,3-dioxan der Formel (IV) ist aus Pishch. Prom-st. (Moscow) 1990, (2), 54-57 bekannt.

3-Methyl-3-penten-2-on der Formel (V) ist aus Perfumer & Flavorist 2001, 26(2), 16-21 bekannt.

Erfindungsgemäße Mischungen, die aus den Bestandteilen (a) und (b) und/oder (c) bestehen, besitzen im Vergleich mit einer Verbindung der Formel (I) eine noch verstärkte Damascon-Note; überdies wirkt der Geruchseindruck einer solchen Mischung insgesamt noch frischer, fruchtiger, strahlender und komplexer als der Geruchseindruck einer Verbindung der Formel (I) per se. Entsprechendes gilt für den Vergleich zwischen Mischungen, die einerseits neben ein, zwei oder mehr weiteren Riechstoffen lediglich eine Verbindung der Formel (I) umfassen und andererseits neben ein, zwei oder mehr weiteren Riechstoffen nicht nur als Bestandteil (a) eine Verbindung der Formel (I) umfassen, sondern daneben auch als Bestandteil (b) und/oder (c) eine Verbindung der Formel (IV) und/oder eine Verbindung der Formel (V).

Eine bevorzugte erfindungsgemäße Mischung umfasst oder besteht aus:
(a) insgesamt 78,0 bis 99,9 Gewichtsteilen einer oder mehrerer der Verbindungen nach einem der Ansprüche 1 bis 5,
   und
(b) 0,25 bis 20 Gewichtsteilen 2-Ethyl-2,5,5-trimethyl-1,3-dioxan der Formel (IV)
   und/oder
(c) 0,1 bis 3,0 Gewichtsteilen 3-Methyl-3-penten-2-on der Formel (V),
wobei die Summe der Bestandteile (a), (b) und (c) 100 Gewichtsteile beträgt.
Daneben sind gegebenenfalls noch ein, zwei oder mehr weitere Riechstoffe vorhanden.

Eine besonders bevorzugte erfindungsgemäße Mischung umfasst oder besteht aus:
(a) 91 bis 99,75 Gewichtsteilen einer oder mehrerer der Verbindungen nach einem der Ansprüche 1 bis 5,
   und
(b) 0,5 bis 8 Gewichtsteilen 2-Ethyl-2,5,5-trimethyl-1,3-dioxan der Formel (IV)
   und/oder
(c) 0,25 bis 1 Gewichtsteilen 3-Methyl-3-penten-2-on der Formel (V),
wobei die Summe der Bestandteile (a), (b) und (c) 100 Gewichtsteile beträgt,
sowie gegebenenfalls ein, zwei oder mehr weiteren Riechstoffen.

Die vorliegende Erfindung betrifft auch die Verwendung einer erfindungsgemäßen Verbindung der Formel (I), bevorzugt also einer Verbindung der Formel (II) und besonders bevorzugt einer Verbindung der Formel (III), wobei die Doppelbindung jeweils besonders bevorzugt (E)-Konfiguration besitzt, als Riechstoff oder Booster.

Entsprechend betrifft die vorliegende Erfindung auch die Verwendung einer erfindungsgemäßen Mischung, welche die oben spezifizierten Bestandteile (a) sowie (b) und/oder (c) sowie gegebenenfalls ein, zwei oder mehr weitere Riechstoffe umfasst, als Riechstoffmischung oder parfümiertes Produkt.

Die Herstellung der erfindungsgemäßen 2-Methyl-2-alkenyl-substituierten 1,3-Dioxane der Formel (I) erfolgt beispielsweise nach allgemein bekannten Methoden der organischen Synthese mittels Umsetzung (Ketalisierung) des entsprechenden Ketons der Formel (K) (mit Resten R² und R³) mit einem entsprechenden aliphatischen 1,3-Diol der Formel (D) (mit Rest R¹), wie nachfolgend schematisch beschrieben: 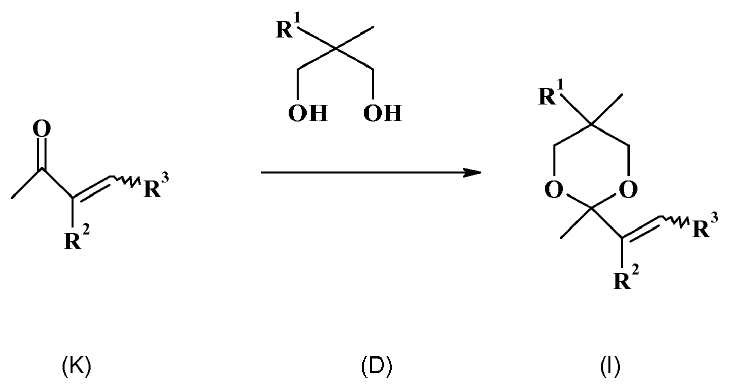 wobei
R¹, R² und R³ jeweils die oben genannte Bedeutung haben, vorzugsweise eine oben als bevorzugt bezeichnete Bedeutung.

Die Konfiguration der Doppelbindung des Ketons (K) bleibt bei Durchführung der Ketalisierung erhalten; bevorzugt ist somit der Einsatz von Ketonen mit (E)-Konfigurationen an der Doppelbindung.

Die Ketalisierung wird vorzugsweise in Gegenwart eines sauren Katalysators, wie beispielsweise p-Toluolsulfonsäure, durchgeführt (vgl. T. Eicher, L.F. Tietze, Organisch-chemisches Grundpraktikum, Georg Thieme Verlag Stuttgart, 1993, 198), alternativ sind aber selbstverständlich auch andere für Ketalisierungsreaktionen eingesetzte Säuren wie Methansulfonsäure oder Schwefelsäure geeignet.

Bei der Ketalisierungsreaktion werden vorzugsweise zumindest 1,2 molare Äquivalente an Diol (D) eingesetzt, bezogen auf das eingesetzte Keton (K). Die Reaktion wird bevorzugt in einem inerten Kohlenwasserstoff, wie beispielsweise Toluol oder Cyclohexan, als Verdünnungsmittel durchgeführt. Die Reaktionstemperatur liegt vorzugsweise im Bereich von 70 bis 140°C. Die Reaktionszeit beträgt dabei regelmäßig 4 bis 20 Stunden.

Die Ketone (K) können, wie im Falle des (vorzugsweise (E)-konfigurierten) 3-Methyl-3-penten-2-ons der Formel (V), kommerziell erworben oder durch Kondensation von 2-Butanon mit dem entsprechendem Aldehyd in Gegenwart von Kaliumhydroxid als Katalysator (z.B. gemäß J. Chem. Soc. (1944), 66, 1517-1519) hergestellt werden.

Die in die Ketalisierungsreaktion eingesetzten 1,3-Diole (D) sind kommerziell erhältlich.

Die vorliegende Erfindung betrifft auch ein parfümiertes Produkt, umfassend
(A) einen festen oder halbfesten Träger
   und
   eine den festen bzw. halbfesten Träger kontaktierende sensorisch wirksame Menge
   einer
   - erfindungsgemäßen Verbindung der Formel (I), vorzugsweise der Formel (II), besonders bevorzugt der Formel (III), besonders vorteilhaft mit (E)-Konfiguration an der Doppelbindung,
      oder
   - erfindungsgemäßen Mischung umfassend die oben genannten Bestandteile (a) sowie (b) und/oder (c) sowie gegebenenfalls ein, zwei oder mehr weitere Riechstoffe,
      oder
(B) eine flüssige Phase
   und
   darin gelöst oder suspendiert oder damit verdünnt eine sensorisch wirksame Menge einer
   - erfindungsgemäßen Verbindung der Formel (I), vorzugsweise der Formel (II), besonders bevorzugt der Formel (III), besonders vorteilhaft mit (E)-Konfiguration an der Doppelbindung
      oder
   - erfindungsgemäßen Mischung umfassend die Bestandteile (a) sowie (b) und/oder (c) sowie gegebenenfalls ein, zwei oder mehr weitere Riechstoffe.

Übliche sonstige Parfümbestandteile, mit denen die erfindungsgemäßen Verbindungen der Formel (I) und die erfindungsgemäßen Mischungen, die zusätzlich die oben genannten Bestandteile (b) und/oder (c) enthalten, vorteilhaft zu einer Riechstoffmischung (= Parfümöl-Komposition) kombiniert werden können, finden sich z.B. in Steffen Arctander, Perfume and Flavor Chemicals, Eigenverlag, Montclair, N.J., 1969; K. Bauer, D. Garbe, H. Surburg, Common Fragrance and Flavor Materials, 4th Edition, Wiley-VCH, Weinheim 2001.

Im Einzelnen seien genannt:
Extrakte aus natürlichen Rohstoffen wie Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos - Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl (engl. turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl; sowie Fraktionen davon, bzw. daraus isolierten Inhaltsstoffen;
Einzel-Riechstoffe aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der aliphatischen Alkohole wie z. B. Hexanol; Octanol; 3-Octanol; 2,6-Dimethylheptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Tridecensäurenitril; 2,12-Tridecensäurenitril; 3,7-Dimethyl-2,6-octadiensäurenitril; 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal; Acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyl-dodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 7-Cyclohexadecen-1-on; 8-Cyclohexadecen-1-on; 6,7-Dihydro-1,1,2,3,3-pentamethyl-4(5H)-indanon; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexen-carbaldehyd;
der cycloaliphatischen Ketone wie z. B. 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,1 0-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der aromatischen Kohlenwasserstoffe wie z. B. Styrol und Diphenylmethan;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B.; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1 ,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

In erfindungsgemäßen Parfümölkompositionen (= Riechstoffmischungen), welche z. B. oben genannte sonstige Parfümbestandteile umfassen, liegt die eingesetzte Menge von 2-Methyl-2-alkenyl-substituierten 1,3-Dioxanen der Formel (I) üblicherweise im Bereich von 0,001 bis 50 Gew.-%, vorzugsweise 0,05 bis 30 Gew.-% und besonders bevorzugt 0,5 bis 20 Gew.-%, bezogen auf die Gesamtmenge der in der Parfümölkomposition enthaltenen Riechstoffe.

2-Methyl-2-alkenyl-substituierte 1,3-Dioxane der Formel (I) enthaltende Parfümölkompositionen (= Parfümöle) können in flüssiger Form, unverdünnt oder mit einem Lösungmittel verdünnt (als parfümiertes Produkt) für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat. Die genannten Lösungsmittel werden im Rahmen des vorliegenden Textes nicht als Riechstoffe aufgefasst.

Für manche Anwendungen ist es vorteilhaft, erfindungsgemäße Verbindungen der Formel (I) enthaltende Parfümöle (Riechstoffmischungen) an einem Trägerstoff adsorbiert einzusetzen, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer; Cellulose-basierende Stoffe, Zucker oder Kunststoffe wie PVC, Polyvinylacetate oder Polyurethane sein.

Für andere Anwendungen ist es vorteilhaft, erfindungsgemäße Verbindungen der Formel (I) enthaltende Parfümöle mikroverkapselt, sprühgetrocknet, als Einschluß-Komplex oder als Extrusions-Produkt einzusetzen und in dieser Form dem zu parfümierenden (Vor-)Produkt hinzufügen.

Die Eigenschaften derart modifizierter erfindungsgemäßer Parfümölkompositionen werden in manchen Fällen durch sogenanntes "Coaten" mit geeigneten Materialien im Hinblick auf eine gezieltere Duftfreisetzung weiter optimiert, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können.

Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Zutaten, mit denen erfindungsgemäße Verbindungen der Formel (I), Mischungen und Parfümölkompositionen kombiniert werden können, sind beispielsweise:
Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

2-Methyl-2-alkenyl-substituierte 1,3-Dioxane der Formel (I) enthaltende erfindungsgemäße Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und - lotionen, Sonnenschutzcremes- und -lotionen, After-sun-cremes und -lotionen, Handcremes und -lotionen, Fußcremes und -lotionen, Enthaarungscremes und - lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Besonders bevorzugte erfindungsgemäße parfümierte Produkte sind Waschmittel, Hygiene- oder Pflegeprodukte, insbesondere im Bereich der Körperpflege, der Kosmetik und des Haushalts.

Begehrt sind ferner - insbesondere für die Parfümierung von tensidhaltigen Formulierungen, wie zum Beispiel für Shampoos, Waschmittel oder Weichspüler - häufig Riechstoffmischungen mit einer damasconigen, fruchtigen und frischen (Kopf)Note, wobei diese gleichzeitig ein ausgeprägtes Blooming (Geruch aus einer wässrigen Tensidlösung) aufweisen sollen.

Die erfindungsgemäßen 2-Methyl-2-alkenyl-substituierten 1,3-Dioxane der Formel (I) und die oben genannten erfindungsgemäßen Mischungen, welche neben einer oder mehreren Verbindungen der Formel (I) zusätzlich die Verbindung der Formel (IV) und/oder (V) umfassen, bewirken insbesondere in Körperpflegemitteln, speziell in Shampoos, und in Haushaltsprodukten, speziell in Wäscheweichspülern, besondere geruchliche Effekte (siehe auch die nachfolgenden Beispiele). Insbesondere vermögen sie eine damasconige, fruchtige und frische (Kopf)Note zu vermitteln und/oder zu verstärken, wobei gleichzeitig ein ausgeprägtes Blooming (Geruch aus einer wässrigen Tensidlösung) beobachtet wird.

Es wurde ferner gefunden, dass die erfindungsgemäßen Verbindungen der Formel (I) sowie die erfindungsgemäßen Mischungen, insbesondere als Bestandteil eines Shampoos oder Wäscheweichspülers, einen hohen Impact und eine hohe Beliebtheit aufweisen.

Durch einen Anteil an einer oder mehreren Verbindungen der Formel (I) an einer Parfumölkomposition, vorzugsweise in einer Gesamtmenge im Bereich von 0,5 bis 4 Gew.-% bezogen auf das Gesamtgewicht der Parfumölkomposition, insbesondere in Parfumölkompositionen für den Einsatz in Shampoo oder Wäschweichspüler, wird der damasconige, fruchtige und frische Charakter im Vergleich zu der Ausgangskomposition deutlich erhöht und gleichzeitig ein ausgeprägtes Blooming erhalten. Bei einem Anteil einer erfindungsgemäßen Mischung in gleicher Menge tritt dieser Effekt weiter verstärkt auf - insbesondere der damasconige Aspekt - und die Parfumölkomposition erhält einen noch komplexeren Geruchseindruck.

Des Weiteren wirken die Verbindungen der Formel (I) und die oben genannten erfindungsgemäßen Mischungen auch als sogenannter Booster oder Enhancer, d.h. sie bewirken eine Verstärkung des Geruchs bzw. der geruchlichen Wahrnehmung von anderen Riechstoffen in Riechstoffmischungen und Parfümkompositionen.

Verbindungen der Formel (I) sowie die oben genannten erfindungsgemäßen Mischungen verstärken in einer Parfumölkomposition für Wäscheweichspüler besonders den frischen Charakter (sehr starker Booster). Für Parfumölkompositionen im Wäscheweich-Bereich ist ein frischer Effekt von großer Bedeutung. Dieser Effekt wird insbesondere wahrgenommen bei Einsatz der Verbindungen der Formel (I) in einer Gesamtmenge von 0,5 Gew bis 4 Gew.-%, bezogen auf das Gesamtgewicht der Parfumölkomposition.

Die folgenden Beispiele erläutern die Erfindung; sofern nicht anders angegeben beziehen sich Anteile und Prozente auf das Gewicht.

### Beispiel 1: Allgemeine Arbeitsvorschrift zur Herstellung der Verbindung der Formel (I)

Es werden 0,31 mol Keton (K) mit 0,37 mol aliphatischem 1,3-Diol (D) in Cyclohexan mit 0,0003 mol para-Toluolsulfonsäure versetzt und für mindestens zwei Stunden am Wasserabscheider unter Rückfluss gekocht. Das Reaktionsgemisch wird sorgfältig zunächst mit 5 Gew.-%iger Natriumcarbonat-Lösung dann mit Wasser gewaschen. Die organische Phase wird über Kaliumcarbonat getrocknet, eingeengt und im Vakuum destilliert.

### Beispiel 1.1: 2,5-Dimethyl-2-(1'-methyl-propenyl)-1,3-dioxan

Gemäß der obigen allgemeinen Arbeitsvorschrift wurde aus 0,31 mol 3-Methyl-3-penten-2-on (Verbindung der Formel (V)) und 0,37 mol 2-Methyl-1,3-propandiol das erfindungsgemäße 2,5-Dimethyl-2-(1'-methyl-propenyl)-1,3-dioxan hergestellt (50% der Theorie, Reinheit: >98% laut GC; Hauptisomer >95 GC-%, Nebenisomer > 3 GC-%).

### Hauptisomer:

¹H-NMR (400 MHz, CDCl₃): δ (ppm) = 0,65 (d, J = 6,68 Hz, 3H), 1,33 (s, 3H), 1,59 -1,61 (m, 3H), 1,66 - 1,69 (m, 3H), 1,99 - 2,10 (m, 1 H), 3,26 - 3,33 (m, 2H), 3,64 - 3,70 (m, 2H), 5,69 (dq, J = 6,19, 1,45 Hz, 1 H).
¹³C-NMR (101 MHz, CDCl₃): δ (ppm) = 12,01, 12,56, 13,47, 28,63, 28,97, 67,39 (2C), 100,91, 123,01, 133,66

### Nebenisomer:

¹H-NMR (400 MHz, CDCl₃): δ (ppm) = 1,16 (d, J = 6,91 Hz, 3H), 1,37 (s, 3H), 1,62 - 1,63 (m, 3H), 1,65 - 1,67 (m, 3H), 1,99 - 2,10 (m, 1 H), 3,47 - 3,52 (m, 2H), 3,87 - 3,92 (m, 2H), 5,69 (dq, J = 6,19, 1,45 Hz, 1 H).
¹³C-NMR (101 MHz, CDCl₃): δ (ppm) = 11,87, 13,38, 15,11, 25,81, 28,73, 66,11 (2C), 100,66, 122,02, 134,75

### Beispiel 1.2: 2,5,5-Trimethyl-2-(1'-methyl-propenyl)-1,3-dioxan

Gemäß der obigen allgemeinen Arbeitsvorschrift wurde aus 0,31 mol 3-Methyl-3-penten-2-on (Verbindung der Formel (V)) und 0,37 mol 2,2-Dimethyl-1,3-propandiol das erfindungsgemäße 2,5,5-Trimethyl-2-(1'-methyl-propenyl)-1,3-dioxan hergestellt (72% der Theorie, Reinheit: >99% laut GC, 1 Peak, nur (E)-Isomer).
¹H-NMR (400 MHz, CDCl₃): δ (ppm) = 0,68 (s, 3H), 1,19 (s, 3H), 1,36 (s, 3H), 1,58 - 1,61 (m, 3H), 1,65 - 1,68 (m, 3H), 3,26 - 3,30 (m, 2H), 3,41 - 3,46 (m, 2H), 5,65 - 5,72 (m, 1 H).
¹³C-NMR (101 MHz, CDCl₃): δ (ppm) = 11,93, 13,45, 22,08, 22,78, 28,05, 29,58, 71,40 (2C) 100,94, 122,73, 133,59.

### Beispiel 2: Parfümöl, z.B. für den Einsatz in Shampoo

Herstellung eines Parfümöles mit einer modernen, ausgeprägt frischen, fruchtigen und damasconigen Note.

Es werden die folgenden Riechstoffe in den angegebenen Mengen vermischt:

| | Gewichtsteile |
|---|---|
| Agrumex (2-tert.-Butylcyclohexylacetat) | 9 |
| Aldehyd C 14 sogenannt | 5 |
| Alkohol C 6 koscher | 2 |
| Allylcapronat koscher 10%ig in DPG | 1 |
| Benzylformiat | 1,5 |
| Butylacetat | 0,5 |
| Dibenzylether | 1,5 |
| Dipropylenglycol | 37,5 |
| Ethylbutyrat | 0,5 |
| Ethyldecadienoat trans, cis-2,4- | 1 |
| Ethylmethylbutyrat -2 | 0,5 |
| Exaltolide | 2 |
| Geranylacetat | 10 |
| Globalide^{®} (11(12)-Pentadecen-15-olid) | 6 |
| Hexenol cis-3 | 0,5 |
| Hexenol trans-2 | 0,5 |
| Hexenylacetat cis-3 | 2 |
| Hexylacetat | 5 |
| Isoamylacetat | 2,5 |
| Ligustral | 0,5 |
| Maltol | 0,5 |
| Nerylacetat | 6 |
| Parmanyl^{®} [3-(cis-3-Hexenyloxy]-propannitril | 1,5 |
| Prenylacetat | 1,5 |
| | |
| Summe | 98,5 |

### DPG: Dipropylenglycol

Diese Ausgangskomposition ist ein Parfümöl mit einem fruchtigen Geruch mit betonter Apfel- und Birnennote.

### Beispiel 2.1:

Durch den Zusatz von 1,5 Gewichtsteilen 2,5,5-Trimethyl-2-(1'-methyl-propenyl)-1,3-dioxan aus Beispiel 1.2 wird ein modernes Parfümöl mit einer ausgeprägt frischen und damasconigen Note erhalten. Im Vergleich zur Ausgangskomposition wird auch der fruchtige Charakter merklich verstärkt, insbesondere wird die birnige Note intensiviert. Die erhaltene erfindungsgemäße Riechstoffkomposition ist harmonischer und abgerundeter, ebenso ist die Ausstrahlung und räumliche Wirkung (Diffusivität) im Vergleich zu der Ausgangskomposition erhöht.

### Beispiel 2.2:

Bei Zusatz von 1,5 Gewichtsteilen einer Mischung bestehend aus 96 Gew.-% 2,5,5-Trimethyl-2-(1'-methyl-propenyl)-1,3-dioxan aus Beispiel 1.2 und 4 Gew.-% 2-Ethyl-2,5,5-trimethyl-1,3-dioxan der Formel (IV) zu der Ausgangskomposition werden die unter Beispiel 2.1 geschilderten Effekte noch verstärkt und das Parfumöl erhält einen noch komplexeren Geruchseindruck.

### Beispiel 3: Shampoo

Die Parfümölkomposition aus Beispiel 2.2 (nach Zusatz von 1,5 Gewichtsteilen 2,5,5-Trimethyl-2-(1'-methyl-propenyl)-1,3-dioxan aus Beispiel 1.2) wurde in einer Dosierung von 0,5 Gew.-% in eine Shampoo-Grundmasse folgender Zusammensetzung eingearbeitet:

| | |
|---|---|
| Natriumlaurylethersulfat (z.B. Texapon NSO, Fa. Cognis Deutschland GmbH) | 12% |
| | |
| Cocamidopropylbetain (z.B. Dehyton K, Fa. Cognis Deutschland GmbH) | 2% |
| | |
| Natriumchlorid | 1,4% |
| | |
| Citronensäure | 1,3% |
| | |
| Phenoxyethanol, Methyl-, Ethyl-, Butyl-, und Propylparaben | 0,5% |
| | |
| Wasser | 82,8% |

Der pH-Wert der Shampoo-Grundmasse lag bei etwa 6. Hieraus wurden 100 mL einer 20 Gew.%-igen wässrigen Shampoo-Lösung hergestellt. Die Shampoo-Lösung zeigte ein ausgeprägtes Blooming. In dieser Shampoo-Lösung wurden 2 Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt. Beide Haarsträhnen zeigten einen fruchtig-frischen, leicht damasconigen Geruch, wobei der Gesamteindruck strahlend, abgerundet und harmonisch empfunden wurde

### Beispiel 4: Parfümöl, z.B. für den Einsatz in Weichspüler

Herstellung eines Parfümöls mit einer modernen, betont blumig-frischen Note.

Es werden die folgenden Riechstoffe in den angegebenen Mengen vermischt:

| | Gewichtsteile |
|---|---|
| Acetophenon 10%ig in DPG | 1 |
| Undecanal | 0,5 |
| Aldehyd C14 sogenannt (Pfirsichaldehyd) | 1,5 |
| Allylamylglycolat 10%ig in DPG | 2 |
| Amylsalicylat n/iso | 2,5 |
| Citronellol | 12 |
| Decenol trans-9 | 0,5 |
| Dihydromyrcenol | 3 |
| Dimethylbenzylcarbinylacetat | 3 |
| Diphenyloxid | 0,5 |
| Eugenol | 1 |
| Geraniol | 6 |
| Geraniumöl ägyptisch | 1,5 |
| Hexenol cis-3, 10%ig in DPG | 0,5 |
| Hexenylsalicylat cis-3 | 2 |
| Indol 10%ig in DPG | 1 |
| Ionon alpha | 1,5 |
| lonon beta | 0,5 |
| Isoeugenol | 0,5 |
| Isoraldein | 1,5 |
| Lilial | 6 |
| Linalool | 4 |
| Methylphenylacetat | 1 |
| Orangenöl brasilianisch | 1 |
| Palmarosaöl | 0,5 |
| Patchouliöl | 0,5 |
| Phenylethylalkohol | 27,5 |
| Rosenoxid 10%ig in DPG | 2 |
| Styrolylacetat | 2 |
| Terpineol | 3 |
| Tetrahydrolinalool | 5 |
| Vanillin 10%ig in DPG | 0,5 |
| Vertocitral | 0,5 |
| Zimtalkohol | 1 |
| | |
| Summe | 97 |

### DPG: Dipropylenglycol

Diese Ausgangskomposition ist ein Parfümöl für Wäscheweichmittel mit einem blumigen Akkord mit wilder Rosennote.

### Beispiel 4.1:

Durch Zugabe von 1,5 Gewichtsteilen 2,5-Dimethyl-2-(1'-methyl-propenyl)-1,3-dioxan aus Beispiel 1.1 und 1,5 Gewichtsteilen 2,5,5-Trimethyl-2-(1'-methylpropenyl)-1,3-dioxan aus Beispiel 1.2 zu der Ausgangskomposition wird ein modernes Parfüm (= erfindungsgemäßes Produkt) mit einer ausgeprägten blumig-frischen Wäscheweich-Note erhalten. Im Vergleich zur Ausgangskomposition wird ein deutlich stärkerer, frischerer blumiger Geruch (sehr starke Verstärkung (Booster)) mit leicht damasconiger Note wahrgenommen. Das erfindungsgemäße Produkt ist harmonischer und abgerundeter, auch wird die Ausstrahlung und räumliche Wirkung (Diffusivität) im Vergleich zu der Ausgangskomposition erhöht.

### Beispiel 4.2:

Bei Zusatz von 1,5 Gewichtsteilen einer Mischung bestehend aus 95,8 Gew.-% 2,5,5-Trimethyl-2-(1'-methyl-propenyl)-1,3-dioxan aus Beispiel 1.2, 3,8 Gew.-% 2-Ethyl-2,5,5-trimethyl-1,3-dioxan der Formel (IV) und 0,4 Gew.-% 3-Methyl-3-penten-2-on der Formel (V) zu der Ausgangskomposition werden die unter Beispiel 4.1 geschilderten Effekte noch verstärkt und das Parfumöl erhält einen noch komplexeren Geruchseindruck.

### Beispiel 5: Weichspüler

Die Parfümölkomposition aus Beispiel 4.1 (nach Zusatz von 2,5-Dimethyl-2-(1'-methyl-propenyl)-1,3-dioxan und 2,5,5-Trimethyl-2-(1'-methyl-propenyl)-1,3-dioxan in den angegebenen Anteilen) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% (z.B. Rewoquat WE 18, Fa. Witco Surfactants GmbH) | 5,5% |
| Alkyldimethylbenzylammoniumchlorid, ca. 50% (z.B. Preventol R50, Fa. Bayer AG) | 0,2% |
| Farblösung, ca. 1%-ig | 0,3% |
| | |
| Wasser | 94,0% |

Der pH-Wert des Weichspüler-Grundmasse lag im Bereich 2 - 3. Zwei Stofflappen wurden mit 370 g einer 1%-igen wässrigen Weichspüler-Lösung in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt. Beide Lappen zeigten einen fruchtig-blumigen, leicht damasconigen Geruch, wobei der Gesamteindruck strahlend, abgerundet und harmonisch empfunden wurde.

## Patentansprüche

1. Verbindung der Formel (I) 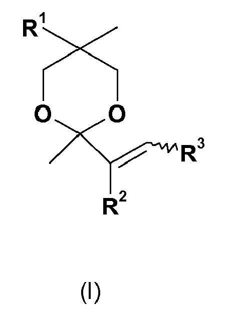 wobei
R¹ Wasserstoff oder Methyl bedeutet, und R² und R³ unabhängig voneinander Methyl oder Ethyl bedeuten.

2. Verbindung nach Anspruch 1, wobei R² Methyl bedeutet.

3. Verbindung nach einem der vorangehenden Ansprüche, wobei R² und R³ jeweils Methyl bedeuten.

4. Verbindung nach einem der vorangehenden Ansprüche, wobei die Doppelbindung (E)-Konfiguration besitzt.

5. Verbindung nach einem der vorangehenden Ansprüche, ausgewählt aus der Gruppe bestehend aus:
(E)-2,5-Dimethyl-2-(1'-methyl-propenyl)-1,3-dioxan
und
(E)-2,5,5-Trimethyl-2-(1'-methyl-propenyl)-1,3-dioxan.

6. Mischung umfassend oder bestehend aus:
(a) einer oder mehreren Verbindungen nach einem der Ansprüche 1 bis 5
und
(b) 2-Ethyl-2,5,5-trimethyl-1,3-dioxan der Formel (IV) 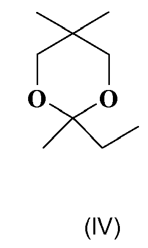 und/oder
(c) 3-Methyl-3-penten-2-on der Formel (V) 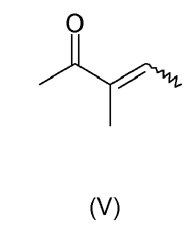
sowie gegebenenfalls ein, zwei oder mehr weiteren Riechstoffen.

7. Mischung nach Anspruch 6, umfassend oder bestehend aus:
(a) insgesamt 78,0 bis 99,9 Gewichtsteilen einer oder mehrerer der Verbindungen nach einem der Ansprüche 1 bis 5,
und
(b) 0,25 bis 20 Gewichtsteilen 2-Ethyl-2,5,5-trimethyl-1,3-dioxan der Formel (IV)
und/oder
(c) 0,1 bis 3,0 Gewichtsteilen 3-Methyl-3-penten-2-on der Formel (V),
wobei die Summe der Bestandteile (a), (b) und (c) 100 Gewichtsteile beträgt, sowie gegebenenfalls ein, zwei oder mehr weiteren Riechstoffen.

8. Mischung nach einem der Ansprüche 6 bis 7, umfassend oder bestehend aus:
(a) insgesamt 91 bis 99,75 Gewichtsteilen einer oder mehrerer der Verbindungen nach einem der Ansprüche 1 bis 5,
und
(b) 0,5 bis 8 Gewichtsteilen 2-Ethyl-2,5,5-trimethyl-1,3-dioxan der Formel (IV) und/oder
(c) 0,25 bis 1 Gewichtsteilen 3-Methyl-3-penten-2-on der Formel (V),
wobei die Summe der Bestandteile (a), (b) und (c) 100 Gewichtsteile beträgt, sowie gegebenenfalls ein, zwei oder mehr weiteren Riechstoffen.

9. Mischung nach einem der Ansprüche 6 bis 8, wobei die eingesetzte Gesamtmenge an Verbindungen nach einem der Ansprüche 1 bis 5 im Bereich von 0,001 bis 50 Gew.-%, vorzugsweise 0,05 bis 30 Gew.-% und besonders bevorzugt 0,5 bis 20 Gew.-% liegt, bezogen auf die Gesamtmenge der in der Mischung enthaltenen Riechstoffe.

10. Verwendung
einer Verbindung nach einem der Ansprüche 1 bis 5 als Riechstoff oder Booster oder
einer Mischung nach einem der Ansprüche 6 bis 9 als Riechstoffmischung oder parfümiertes Produkt.

11. Verfahren zur Herstellung einer Verbindung nach einem der Ansprüche 1 bis 5, mit folgenden Schritten: Umsetzen eines Ketons der Formel (K) 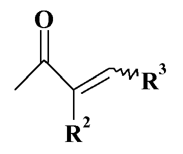 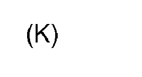 wobei R² und R³ jeweils die in einem der Ansprüche 1 bis 5 genannte Bedeutung haben,
mit einem aliphatischen 1,3-Diol der Formel (D) 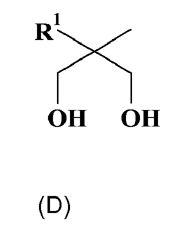 wobei R¹ die in einem der Ansprüche 1 bis 5 genannte Bedeutung hat,
vorzugsweise in Gegenwart eines sauren Katalysators.

12. Parfümiertes Produkt, umfassend
(A) einen festen oder halbfesten Träger
und
eine den festen bzw. halbfesten Träger kontaktierende sensorisch wirksame Menge einer
- Verbindung nach einem der Ansprüche 1 bis 5
oder
- Mischung nach einem der Ansprüche 6 bis 9,
oder
(B) eine flüssige Phase
und
darin gelöst oder suspendiert oder damit verdünnt eine sensorisch wirksame Menge einer
- Verbindung nach einem der Ansprüche 1 bis 5
oder
- Mischung nach einem der Ansprüche 6 bis 9.

13. Parfümiertes Produkt nach Anspruch 12, ausgewählt aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, saure, alkalische und neutrale Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und - lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

14. Parfümiertes Produkt nach Anspruch 12 oder 13, ausgewählt aus der Gruppe bestehend aus: Shampoos und Wäscheweichspüler.

15. Verfahren zum Vermitteln, Modifizieren und/oder Verstärken eines Geruches, mit folgendem Schritt:
Kontaktieren oder Vermischen eines Erzeugnisses mit einer sensorisch wirksamen Menge einer
- Verbindung nach einem der Ansprüche 1 bis 5
oder
- Mischung nach einem der Ansprüche 6 bis 9.
